Europäisches Patentamt

European Patent Office

Office européen des brevets

(11) Numéro de publication: **0 312 131**
**A2**

## (12) DEMANDE DE BREVET EUROPEEN

(21) Numéro de dépôt: **88201396.4**

(22) Date de dépôt: **21.06.88**

(51) Int. Cl.⁴: **A61M 5/00 , B65F 1/14**

(30) Priorité: **22.06.87 FR 8708901**

(43) Date de publication de la demande:
**19.04.89 Bulletin 89/16**

(84) Etats contractants désignés:
**ES GR**

(71) Demandeur: **Germain, Bruno**
**1, rue de l'Eglise**
**F-69650 Saint-German au Mont d'Or(FR)**

(72) Inventeur: **Germain, Bruno**
**1, rue de l'Eglise**
**F-69650 Saint-German au Mont d'Or(FR)**

(74) Mandataire: **Eggert, Hans-Gunther, Dr.**
**Räderscheidtstrasse 1**
**D-5000 Köln 41(DE)**

(54) **Dispositif monocoque pour le démontage et le stockage étanche d'aiguilles medicales usagées.**

(57) Dispositif portatif monocoque pour le démontage et le stockage étanche d'aiguilles hypodermiques usagées et équivalentes, en vue de leur destruction ultérieure, qui comprend
: - Un boitier-réceptacle (1) muni d'un orifice d'accès (2) pour les aiguilles.
- A l'intérieur du boitier-réceptacle (1), et au-dessous de l'orifice d'accès (2) un écran plan mobile (3) qui obstrue le dit orifice et le libère à la demande par déplacement.
- Un organe de commande (4) de l'écran (3) muni de moyens de liaison (10) et de rappel (11).

Le dispositif est d'un maniement aisé, et à fermeture automatique.

Il est mis en oeuvre dans tous les lieux où l'on utilise des aiguilles hypodermiques ou équivalentes pour assurer la protection du personnel médical, ou toutes personnes risquant d'être confrontées aux risques de blessures par aiguilles usagées et/ou contaminées, et risquant par conséquent de contracter une infection grave, et accidentelle. (Hépatites B, virale, SIDA, etc...)

Les applications de ce dispositif sont axées en milieu hospitalier, cliniques, laboratoires d'analyses médicales, cabinets dentaires et vétérinaires, centres de soins, dispensaires, sanatorium, maisons de retraites, cabinets médicaux, infirmières en clientèle, centres de transfusion sanguine, SAMU, centres de secours sapeurs pompiers, Instituts de Recherches Médicales, etc...

## Dispositif Monocoque Pour Le Demontage Et Le Stockage Etanche d'Aiguilles Medicales Usagees

L'invention concerne un dispositif monocoque pour le démontage et le stockage étanche d'aiguilles hypodermiques usagées ou équivalentes en vue de leur destruction.

Depuis longtemps le milieu médical, dentaire, vétérinaire, utilise dans les hopitaux, cliniques, laboratoires d'analyses et tous autres lieux de soins et appropriés, des aiguilles hypodermiques ou équivalentes, telles que canules ou trocards pour prélever des liquides à analyser tel que sang ou pour administrer des médicaments ou autres produits de traitements médicaux.

Ces aiguilles, dont il est fait une consommation importante, sont mises à la disposition du personnel médical sous forme individuelle ensachée et stérilisée. Après un premier usage, les aiguilles ne sont pas restérilisées, mais sont détruites ou stockées en vue de leur destruction.

L'opération de stockage en vue de la destruction des aiguilles hypodermiques ainsi usagées et contaminées ou équivalentes, doit s'accomplir d'une manièré telle que le personnel médical, ou autres personnes présentes ne risquent pas une contamination par blessure accidentelle et que les aiguilles usagées et stockées ne soient plus accessibles pour un ré-emploi par des consommateurs de substances interdites et dangereuses.

C'est pourquoi, il s'est révèlé souhaitable que la séperation de l'aiguille et de la seringue se fasse sans contact manuel pour éviter tous risques de blessures accidentelles et tous risques de contamination qui en résultent.

C'est pourquoi il s'est révèlé également souhaitable, d'isoler l'aiguille usagée immédiatement après sa séparation d'avec la seringue afin d'éviter tout contact humain, accidentel ou intentionnel, qui pourait conduire à une contamination ultérieure.

Pour ce faire, plusieurs types de dispositifs ont été proposés pour faciliter la manipulation de l'aiguille et son stockage après sa séparation d'avec la seringue.

Un premier type de dispositif dont un exemple est décrit dans le brevet FR 2,586,566 consiste en un support destiné à recevoir le fourreau protecteur d'une aiguille hypodermique stérile. Ce dispositif facilement préhensible, est formé d'un corps léger cylindrique doté selon son axe d'un réceptacle à commande bloquante pour le fourreau protecteur de l'aiguille, et, à l'extrêmité donnant accès au réceptacle, d'une corolle protectrice de la main tenant le corps léger cylindrique. Ainsi l'aiguille hypodermique usagée est réintroduite dans son fourreau protecteur (placé en position bloquée dans le dispositif) sans risque de blessure pour l'opérateur médical tenant le dispositif.

Ce dispositif présente des inconvénients, dont deux au moins semblent majeurs.

Un premier inconvénient réside dans le fait que le dispositif exige deux manoeuvres pour l'opérateur médical, l'une étant la mise en place du fourreau contenant l'aiguille hypodermique stérile, puis l'extraction de l'aiguille stérile de son fourreau, l'autre étant la réintroduction de la dite aiguille usagée dans le fourreau, en utilisant la corolle protectrice de la main tenant le dispositif, pour guider l'aiguille sans risque de se blesser.

Un autre inconvénient plus grave, apparait à l'esprit quand il est perçu que le dispositif n'élimine pas l'aiguille usagée, et ne permet que sa ré-introduction dans le fourreau, et dès lors en laisse l'accès possible et facile avec tous les risques de blessures et d'insécurité, et de contamination évidente.

Un autre type de dispositif concerne les appareils destinés à tronquer les aiguilles hypodermiques usagées et à en recueillir les fragments dans leur partie basse munie d'un conteneur amovible.

Un tel dispositif est décrit, par exemple, dans le brevet US 4,255,996. Dans cette publication, le dispositif est formé de deux parties, l'une haute, l'autre basse, s'emboitant l'une dans l'autre pour constituer une enceinte close, mais démontable. La partie haute est munie d'un orifice circulaire d'accès pour l'aiguille usagée, d'un diamètre supérieur à l'embout de la dite aiguille, orifice complété par une fente de direction radiale permettant la retenue verticale de l'aiguille usagée, en position de coupe par l'appui de l'embout sur les bords de la dite fente. La partie haute est également munie d'un mécanisme de coupe comportant deux lames parallèles pivotantes dotées d'une commande manuelle externe. La partie basse est équipée d'un réceptacle amovible recueillant les fragments d'aiguilles usagées tronquées.

Toutefois, ce dispositif intéressant par les résultats qu'il procure, présente aussi des inconvénients dont certains peuvent être considérés comme importants. Un inconvénient premier, se manifeste par l'obligation pour l'opérateur médical d'avoir un contact manuel avec l'aiguille usagée, voire contaminée, pour la séparer de la seringue, avant ou après qu'elle ait été tronquée.

Un autre inconvénient se manifeste dès lors que l'orifice d'accès peut rester ouvert après la coupe et que l'enceinte est ouvrable par la séparation des parties haute et basse la formant. Ainsi le dispositif destiné à tronquer les aiguilles hypodermiques usagées n'est pas étanche et rend possible l'accès aux aiguilles tronquées avant leur destruction ultérieure, par le feu, par exemple. De plus, leur destruction en particules n'exclue par l'infection de celles-ci, d'autant qu'il faille éliminer ces particules infectées en les vidant dans un autre récipient. L'isolation n'est donc pas parfaite à ce titre.

Un dernier type de dispositif concerne ceux dont la vocation est de permettre la séparation de l'aiguille hypodermique usagée d'avec la seringue, son isolement immédiatement après la séparation en évitant tout B0 contact humain accidentel qui pourrait provoquer une contamination de l'operateur médical.

Un tel dispositif a été décrit, par exemple, dans le PCT WO 82/00412, et consiste en un conteneur cylindrique dont la paroi supérieure est munie d'un orifice circulaire de diamètre inférieur à celui de l'embout de l'aiguille hypodermique. Cet orifice est placé à l'intersection de deux fentes en croix qui créent dans la paroi supérieure une zone flexible. L'aiguille usagée solidaire de la seringue est introduite en force dans l'orifice jusqu'à ce que l'embout traverse la paroi supérieure. Par un traction de la seringue, l'aiguille est libérée et tombe au fond du conteneur.

Mais ce dispositif présente aussi des inconvénients importants.

L'un d'entre eux est constitué par le fait que l'orifice est en état d'ouverture permanent, rendant possible ou bien l'accès aux aiguilles usagées, ou encore le risque que certaines d'entre elles s'en échappent lors de la manipulation du conteneur.

Un autre inconvénient se manifeste à travers la possible déformation des fentes à l'usage, ne permettant plus la traction pratiquée sur la seringue pour assurer la séparation d'avec l'aiguille usagée.

Un dernier inconvénient est lié à la forme de l'orifice en fentes qui ne permet pas l'arrachage des aiguilles de type Vacutainer dont l'embout cannelé est vissé sur le corps de la seringue nécessitant, dès lors, un moyen de blocage pour assurer la séparation de l'aiguille et de la seringue par dévissage.

Un autre dispositif, relevant de ce dernier type décrit dans la demande européenne de Brevet EP 0,123,247, consiste en un récipient collecteur d'aiguilles hypodermiques usagées, en forme de flacon, dont l'ouverture est munie d'une coiffe ayant un orifice de forme triangulaire très spéciale pour permettre l'engagement de l'aiguille solidaire de la seringue, la séparation par traction de la seringue et la chute de l'aiguille.

Dès lors que l'aiguille a été séparée et stockée dans le récipient collecteur, l'orifice triangulaire est aveuglé par un mouvement de rotation de la coiffe, interdisant l'accès du récipient collecteur d'aiguilles.

Bien que ce dispositif évite apparement le contact manuel avec l'aiguille au cours de sa séparation, il apparait être doté de certains inconvénients majeurs qui en limitent l'usage.

Un premier inconvénient réside dans le fait que ce dispositif n'est pas aisément utilisable quand il n'est pas à poste fixe, car l'ouverture de l'orifice triangulaire d'accès s'effectuant par rotation de la coiffe, provoque également la rotation du dispositif s'il n'est pas solidement maintenu en position.

Dés lors, le personnel médical se trouve confronté à deux alternatives. Ou bien se déplacer du patient jusqu'au dispositif de stockage à poste fixe, la seringue munie de son aiguille infectée ou usagée dans une main, risquant ainsi tout accident ou blessure pour lui-même ou un tiers à proximité, l'autre main saisissant la coiffe, lui imprimant un mouvement de rotation jusqu'à l'ouverture de l'orifice triangulaire d'accès.

Ou bien, le manipulateur pose la seringue munie de l'aiguille usagée, saisit à deux mains le dispositif de stockage, l'un entrainant la rotation de la coiffe, l'autre bloquant le dispositif.

Un autre inconvénient se manifeste dans le fait que l'orifice d'accès de forme triangulaire ne s'adapte pas au diamètre de l'embout de l'aiguille à séparer de la seringue. De ce fait, pour que l'aiguille soit retenue par le dit orifice, le manipulateur, après introduction de l'aiguille solidaire de la seringue dans la zone la plus large de l'orifice triangulaire, déplace la seringue selon une translation horizontale jusqu'à ce que l'aiguille soit bloquée par le rétrécissement de l'orifice triangulaire. .

Il en découle que l'on ne l'eut éviter le blocage occasionnel d'une aiguille dans l'orifice triangulaire nécessitant son dégagement manuel, avec risques de blessures et de contamination.

Un troisième inconvénient réside dans le volume du dispositif qui le rend relativement encombrant et d'un transport peu aisé puisqu'il doit être assujetti à un poste fixe, tel qu'une table roulante de soins, par exemple, ou dans une salle de soins.

A noter pour ce type de dispositif, qu'il ne répond pas là aussi à tous les types d'aiguilles, en particulier les aiguilles types cacutainer, cataiter, épicranienne, montées par vissage sur les seringues, et ne pouvant par conséquent être désolidarisées du corps de la seringue, que par dévissage.

Cependant, il existe un réel besoin pour un dispositif amélioré, étanche, pratique, peu encombrant, de caractéristiques dimensionnelles restreintes pour être facilement transportable, maniable sans risque, mis dans une poche, tenant dans la main, dans lequel l'aiguille usagée puisse être introduite facilement, sans risque, et séparée de la seringue sans contact humain direct, et dans lequel l'aiguille collectée est définitivement isolée.

Forte des inconvénients précités et des exigences de sécurité, de prévention, d'hygiène pour le personnel soignant, de même que pour les malades et de tout le personnel d'hôpitaux, cliniques, cabinets, etc, maniant des aiguilles hypodermiques usagées et contaminées, ou équivalentes, l'invention répond à ces exigences et apporte d'autres avantages.

Le dispositif selon l'invention, pour le démontage et le stockage étanche d'aiguilles hypodermiques usagées ou équivalentes en vue de leur destruction, comprend:
- Un boitier-réceptacle muni d'un orifice d'accès pour les aiguilles.
- A l'intérieur de boitier-réceptacle et au-dessous de l'orifice d'accès, un écran plan mobile qui obture le dit orifice mais qui le libère à la demande par déplacement.
- Un organe de commande de l'écran mobile.

Le boitier-réceptacle au contraire des dispositifs de l'art antérieur, a des dimensions telles qu'il est aisément placé dans une poche de blouse, facilement appréhendé par la main libre de l'opérateur médical et tenu en position stable pendant que l'autre main tient la seringue munie de l'aiguille usagée à éliminer.

Le boitier-réceptacle dont la section peut être indifféremment polygonale ou circulaire est du type monocoque, c'est-à-dire que tous les moyens nécessaires au fonctionnement du dispositif y sont enfermés. Ce boitier est généralement constitué de deux demi-coquilles moulées qui permettent avant leur assemblage d'introduire tous les moyens intervenant dans la réalisation et le fonctionnement du dispositif selon l'invention.

L'assemblage ultérieur des deux coquilles par soudage rend le boitier-réceptacle parfaitement étanche.

L'orifice d'accès, dont est muni le boitier-réceptacle, est généralement équipé d'une pièce tronconique externe servant de guide pour l'aiguille hypodermique usagée, et de butée pour la seringue.

La périphérie interne de l'orifice d'accès peut être dotée en tout ou partie de cannelures ou dents, qui favorisent la préhension et le blocage de l'embout de l'aiguille, plus particulièrement quand celle-ci est fixée à la seringue grâce à un pas de vis, fréquents chez les vacutainer, cataiter, et permettant un dévissage aisé, sans effort. A fortiori quand il s'agit d'aiguilles hypodermiques fixées en force sur le corps de la seringue, ou la préhension est excellente.

A l'intérieur du boitier-réceptacle, dans l'axe et au-dessous de l'orifice d'accès, est placé un écran plan mobile, dont le mouvement de translation ou de rotation est commandé de l'extérieur du boitier par l'opérateur médical. Cet écran obturant l'orifice d'accès quand il est au repos.

L'écran plan mobile est généralement formé d'une lame unique rigide pouvant comporter un évidement de forme géomètrique telle que de préférence carrée, rectangulaire, circulaire, hémicirculaire, hémiélliptique ou autres, élliptique.

D'une manières très préférentielle, l'évidement est de forme circulaire, dispose d'un diamètre au moins égal à celui de l'orifice d'accès et est placé en concordance axiale avec lui quand l'écran plan mobile, mis en mouvement, libère l'orifice d'accès.

De même que l'orifice d'accès, l'évidement de l'écran plan mobile peut être doté en tout ou partie de cannelures ou dents facilitant la séparation de la seringue et de l'aiguille à embout vissant usagée, par le blocage de cette dernière, la séparation étant d'autant plus aisée que l'orifice et l'évidement sont simultanément munis de ces cannelures ou dents.

Selon une variante, l'écran mobile obturant l'orifice d'accès est formé de plusieurs lamelles croisées constituant un obturateur de type IRIS.

L'écran mobile est rendu solidaire d'un organe de commande de son mouvement provoqué par le contact avec l'un des doigts de la main tenant le dispositif selon l'invention.

Dès lors qu'il est en mouvement, l'écran plan mobile libère l'orifice d'accès permettant l'introduction de l'aiguille hypodermique usagée dans le boitier-réceptacle.

L'organe de commande de l'écran plan mobile est muni d'un moyen de rappel dans la position d'obturation de l'orifice d'accès quand il est libéré de contact digital, rendant automatique et sécurisante la fermeture du dispositif, après usage.

Enfin, le dispositif selon l'invention peut être doté à l'intérieur du boitier-réceptacle, de moyens antiretour empêchant définitivement l'accès même accidentel, aux aiguilles hypodermiques usagées et stockées, et rendant étanche le dit boitier en y maintenant les liquides provenant des aiguilles démontées, même lors d'un retournement du boitier réceptacle et de l'ouverture intempestive de l'orifice.

Ces moyens antiretours sont constitués par exemple, d'écrans fixes de formes planes, tronconiques, troncpyramidale, munis d'un orifice de section circulaire, polygonale, elliptique ou autres, éventuellement dotés d'un embout tubulaire, cylindrique, tronconique, troncpyramidale ou autres, permettant l'introduction de l'aiguille usagée et interdisant son retour.

Les matériaux mis en oeuvre pour la réalisation du dispositif selon l'invention sont en général réalisés en matières polymères thermo-formables, tels que, par exemple, les polyolifines, les polyamides, polyesters, les polymères halogènés.

Ces matières polymères peuvent recevoir des charges constituées par des matériaux minéraux pulvérulents, tels que carbonate de calcium, sulfate de calcium, kaolins, dioxyde de titane, talc, alumine, mais aussi par des fibres minérales et/ou organiques, telles que par exemple, fibres de verre, fibres céramiques, fibres de carbone.

Ces matières polymères peuvent également recevoir des adjuvants très divers, tels que par exemple, des colorants, des agents bioxydes, des agents antiphotons, etc...

Aussi, et contrairement à l'art antérieur, le dispositif selon l'invention présente de multiples avantages grâce à la conception du boitier-réceptacle monocoque. Bien plus, sa forme et ses dimensions permettent à l'opérateur médical de le manier aisément d'une main tandis que l'autre tient la seringue munie de l'aiguille usagée.

Enfin, le retour automatique dans la position d'obturation de l'orifice d'accès après l'introduction de l'aiguille hypodermique usagée ou équivalente évite l'ultime geste de sécurité que le personnel médical peut omettre quand de tels dispositifs doivent être clos manuellement.

L'invention sera mieux comprise grâce à la description illustrative et non limitative faite à partir des dessins annexés.

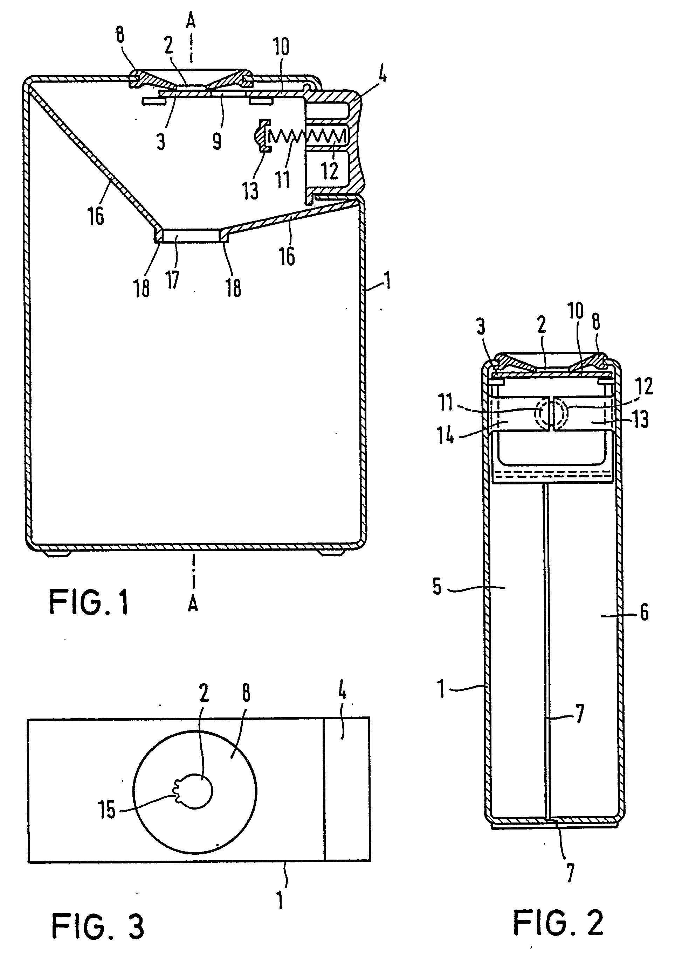

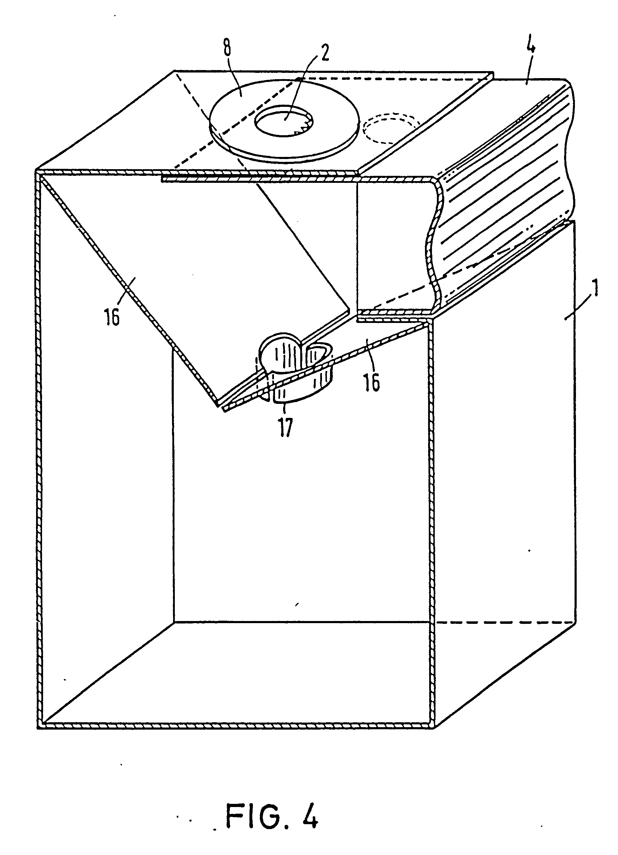

La figure (1) est une coupe longitudinale en élévation d'un dispositif réalisé selon l'invention.

La figure (2) est une coupe transversale selon AA du même dispositif selon l'invention.

La figure (3) est une vue par dessus de l'orifice muni de cannelures.

Selon les figures (1) et (2), le dispositif pour le démontage et le stockage étanche d'aiguilles hypodermiques usagées ou équivalentes se compose d'un boitier-réceptacle (1) muni d'un orifice d'accès (2). Au-dessous de l'orifice d'accès (2) et à l'intérieur du boitier-réceptacle (1), est placé un écran plan mobile (3) et un organe de commande (4) du dit écran mobile.

Le boitier-réceptacle (1) est formé de deux demi-coquilles moulées (5) et (6) assemblées par soudage en fin de montage selon la ligne (7).

L'orifice d'accès (2) est muni d'une pièce tronconique externe (8) servant de guide pour l'aiguille usagée et de butée pour la seringue.

L'écran plan mobile (3) est muni de l'évidement (9) de forme circulaire de même diamètre que celui de l'orifice (2).

Le dit écran (3) est rendu solidaire du poussoir (4), organe de commande du mouvement, par l'intermédiaire du moyen de liaison (10).

L'organe de commande de l'écran plan mobile (3) constitué par le poussoir (4) et le moyen de liaison (10) est muni d'un moyen de rappel (11), constitué par un ressort de compression, dont l'une des extrémités est placée dans le logement (12) du poussoir, tandis que l'autre extrémité prend appui sur les butées (13) et (14) solidaires des demi-coquilles (5) et (6).

Au-dessous de l'écran plan mobile (3) est placé un moyen antiretour formé par les écrans plans fixes (16) doté d'un orifice (17) coaxiale à l'orifice d'accès (2) du boitier-réceptacle (1) et d'un embout tubulaire (18).

Selon la figure (3), l'orifice d'accès (2) du boitier-réceptacle (1) est muni de la pièce tronconique externe (8) servant de guide pour l'aiguille usagée et de butée pour la seringue.

La périphérie interne de l'orifice d'accès (2) est dotée de trois cannelures ou dents (15) qui favorisent le blocage de l'aiguille usagée en vue de faciliter sa séparation d'avec la seringue, et en particulier d'avec les aiguilles fixées par vissage sur le corps de la seringue (vacutainer et cataiter, épicranienne).

Cette pièce (15) cannelée ou crantée marque l'originalité de préhension pour tous types d'aiguilles fixées à la seringue, soit en force, soit par vissage, et facilite les séparations des aiguilles du corps de la seringue, sans aucun risque de contact avec les doigts de l'opérateur médical ou tout personnel soignant.

En pratique, le dispositif selon l'invention tel que décrit, est mis en oeuvre de la manière suivante:

- L'opérateur médical tenant d'une main la seringue munie de l'aiguille hypodermique usagée, saisit le dispositif de l'autre main et, d'un doigt de cette dernière, fait pression sur le poussoir (4). Le mouvement est alors transmis par le moyen de liaison (10) à l'écran plan mobile (3). L'évidement circulaire (9) est, de ce fait, placé en concordance avec l'orifice d'accès (2).

- L'aiguille hypodermique usagée est alors introduite dans l'orifice d'accès (2) et traverse l'évidement circulaire (9) de l'écran plan mobile (3). La seringue portant l'aiguille usagée vient en contact de la pièce tronconique (8).

- L'opérateur médical relâche alors la pression du poussoir (4).

- L'évidement circulaire (9) sous l'action du ressort (11) tend à revenir à sa position initiale, et de ce fait, bloque l'embout de l'aiguille, embout en force ou embout cannelé pris par les cannelures ou dents, qui est aisément séparé de la seringue, soit en force, soit en accusant des tours pour pratiquer le dévissage de l'aiguille usagée pour la dégager du corps de la seringue s'il s'agit d'un type d'aiguille à embout vissant. Dans tous les cas, l'aiguille usagée se sépare aisément par l'effet d'une simple traction. L'aiguille hypodermique usagée tombe alors dans le boitier-réceptacle (1).

L'écran plan mobile (3) libéré, revient à sa position initiale sous l'action du ressort (11) et obture ainsi l'orifice d'accès (2) isolant hermétiquement les aiguilles usagées dans le boitier-réceptacle et sans risque que ces aiguilles puissent ressortir du dit boitier-réceptacle, garantissant une réelle sécurité.

## Revendications

1. Dispositif portatif monocoque pour le démontage et le stockage étanche d'aiguilles hypodermiques usagées et équivalentes, en vue de leur destruction ultérieure, qui comprend:
- Un BOITIER-RECEPTACLE (1) muni d'un ORIFICE d'ACCES (2) pour les aiguilles.
- A l'intérieur du boitier-réceptacle (1) et au-dessous de l'orifice d'accès (2), un ECRAN PLAN MOBILE (3) qui obture le dit orifice et le libère à la demande p'ar déplacement.
- Un ORGANE DE COMMANDE (4) du mouvement de l'écran (3).

2. Dispositif selon la revendication (1) caractérisé par le fait que l'orifice d'accès (2) est muni en tout ou partie de cannelures ou dents.

3. Dispositif selon les revendications (1) et (2) caractérisé par le fait que l'orifice d'accès (2) est muni d'une pièce tronconique externe, de guidage de l'aiguille hypodermique usagée, et de butée pour la seringue solidaire de l'aiguille.

4. Dispositif selon l'une quelconque des revendications (1) à (3) caractérisé en ce que l'écran plan mobile (3) est formé d'une lame unique rigide.

5. Dispositif selon l'une quelconque des revendications (1) à (4) caractérisé en ce que l'écran plan mobile (3) comporte un évidement (9) ayant préférentiellement une forme carrée, rectangulaire, circulaire, hémicirculaire, élliptique, ou hémiélliptique.

6. Dispositif selon la revendication (5) caractérisé en ce que la plus petite dimension de l'évidement (9) est au moins égale au diamètre de l'orifice d'accès (2).

7. Dispositif selon l'une quelconque des revendications (1) à (6) caractérisé en ce que l'évidement (9) de l'écran plan mobile (3) est muni en tout ou partie de cannelures ou dents.

8. Dispositif selon la revendication (1) caractérisé en ce que l'écran plan mobile (3) est formé de plusieurs lamelles croisées constituant un obturateur de type IRIS.

9. Dispositif selon l'une quelconque des revendications (1) à (8) caractérisé en ce que l'organe (4) de commande de l'écran (3) est un poussoir, le dit organe étant muni d'un moyen de liaison (10) avec l'écran plan mobile (3) et d'un moyen de rappel (11).

10. Dispositif selon la revendication (9) caractérisé en ce que le moyen de rappel est un ressort.

11. Dispositif selon l'une quelconque des revendications de (1) à (10) caractérisé en ce que le boitier-réceptacle (1) est muni d'un moyen antiretour (16).

12. Dispositif selon la revendication (11) caractérisé en ce que le moyen antiretour est formé d'écrans fixes de formes planes, tronconiques, troncpyramidales.

13. Dispositif selon l'une des revendications (11) et (12) caractérisé en ce que le moyen antiretour est muni d'un orifice (17).

14. Dispositif selon la revendication (13) caractérisé en ce que l'orifice (17) est doté d'un embout tubulaire (18).

88201396.4

FIG. 1

FIG. 3

FIG. 2

FIG. 4